# EUROPEAN PATENT SPECIFICATION

(11) **EP 2 554 166 B1**
(45) Date of publication and mention of the grant of the patent: **28.08.2019**
(21) Application number: 11762621.8
(22) Date of filing: 22.03.2011
(51) Int. Cl.: A23L 33/115, A61K 9/00, A61K 9/08, A61K 31/23

(54) **OIL AND FAT COMPOSITION FOR THE TREATMENT OF DIABETES**
ÖL- UND FETTZUSAMMENSETZUNG ZUR BEHANDLUNG VON STOMATITIS
COMPOSITION D'HUILE ET DE GRAISSE DESTINÉE À TRAITER LE DIABÈTE

(30) Priority: 28.12.2010 JP 2010293961; 31.03.2010 JP 2010081501
(43) Date of publication of application: 06.02.2013
(73) Proprietor: The Nisshin OilliO Group, Ltd., Tokyo 104-8285 (JP)
(72) Inventor: AOYAMA, Toshiaki, Yokosuka-shi Kanagawa 239-0832 (JP); TERADA, Shin, Yokosuka-shi Kanagawa 239-0832 (JP); YAMAMOTO, Sayuri, Yokosuka-shi Kanagawa 239-0832 (JP)
(74) Representative: EP&C
(86) International application number: PCT/JP2011/056710
(87) International publication number: WO 2011/122389

(56) References cited:
- EP-A1- 1 481 675
- EP-A1- 2 671 579
- WO-A1-2004/022051
- WO-A1-2011/024827
- JP-A- 6 092 848
- JP-A- 2000 509 258
- JP-A- 2002 188 096
- JP-A- 2002 188 096
- JP-A- 2002 199 844
- JP-A- 2007 236 348
- US-A1- 2004 151 757
- US-A1- 2004 191 391
- DENG, BIN ET AL.: 'Effects of medium chain triglyceride on insulin resistance in type 2 diabetes mellitus' ZHONGHUA LINCHUANG YINGYANG ZAZHI vol. 17, no. 3, 2009, pages 148 - 152, XP008162801

## Description

### TECHNICAL FIELD

The present invention relates to an oil or fat composition for the treatment of diabetes to improve insulin resistance the composition containing a certain specific triacylglycerol as an active ingredient.

### BACKGROUND ART

Diabetes is classified broadly into type I diabetes (insulin-dependent diabetes) and type II diabetes (noninsulin-dependent diabetes). The majority of diabetic patients in Japan suffer from type II diabetes, and the disease develops through the progress of conditions such as insulin secretion failure that causes a decrease in the amount of insulin secretion, and insulin resistance that causes an impairment of an action of insulin of lowering a blood glucose level (impaired insulin action). These conditions are significantly related to genetic factors, as well as environmental factors such as unbalanced diet, excessive eating, insufficient exercise and stress.

In recent years, the number of patients with type II diabetes has increased, and already reportedly reaches twenty million including potential diabetic patients who may be referred to as borderline. Therefore, not only care of diabetic patients but also prevention of transition from potential diabetic patients to diabetic patients has been an important issue.

In general, a treatment of type II diabetes includes a pharmacological therapy carried out on the basis of an alimentary therapy and an exercise therapy. In pharmacological therapies for type II diabetes in Japan, a sulfonylurea agent (SU agent) that promotes insulin secretion has been frequently used because Japanese people have an insulin secretory capacity lower than that of Westerners. Also, for conditions of insulin resistance, a thiazolidine type drug, generally referred to, such as pioglitazone is used.

However, an SU agent is accompanied by problems of side effects such as low blood glucose, pruritus, rash, body weight gain and exhaustion of pancreatic β cells, and a thiazolidine type drug is contraindicated for patients suffering from hepatic impairment or renal dysfunction, with involvement of problems of side effects such as edema and body weight gain. Since it is necessary to perform a pharmacological therapy for diabetes consecutively for a long period of time, development of an antidiabetic drug for which a side effect is not concerned even if dosed on a continuous basis has been desired.

As such antidiabetic drugs, a promoting agent of insulin secretion containing an extraction liquid obtained from a skin portion of tuberous root of Ipomoea Batatas sp. as an active ingredient (Patent Document 1), and insulin-sensitizing agents containing acetic acid, β-conglycinin, and γ-aminobutyric acid, respectively, as an active ingredient (Patent Documents 2 to 4) have been reported so far.
Patent Document 1: Japanese Patent Publication No. 3677007
Patent Document 2: Japanese Unexamined Patent Application, Publication No. 2002-193797
Patent Document 3: PCT International Publication No. WO2004-087199
Patent Document 4: Japanese Unexamined Patent Application, Publication No. 2008-74734.

US 2004/0191391 discloses an oil or fat composition that relates to low body fat accumulation. JP 2002188096 discloses a glyceride having an inhibitory function on fat storage.

### DISCLOSURE OF THE INVENTION

The invention is as set out in the claims.

### Problems to be Solved by the Invention

The present invention was made in view of the foregoing circumstances, and an object of the invention is to provide an oil or fat composition that is highly safe, and is effective for the prevention or treatment of diabetes without concern over side effects, even if ingested on a continuous basis, and food or drink that include the same.

### Means for Solving the Problems

The present inventor thoroughly investigated in order to solve the foregoing problems, and consequently found that an oil or fat composition containing as an active ingredient triacylglycerol that includes a medium-chain fatty acid and a long-chain fatty acid each having a specific number of carbon atoms in its molecule as constituent fatty acids can solve the problems described above. Accordingly the present invention was completed.

Specifically, aspects of the present disclosure provide the followings.

According to a first aspect of the present disclosure, an oil or fat composition for treatment of diabetes to improve insulin resistance is characterized by including triacylglycerol as an active ingredient, and the triacylglycerol having one or two medium-chain fatty acid residues per molecule and including as constituent fatty acids a C₆₋₁₀ medium-chain fatty acid and a C₁₆₋₂₄ long-chain fatty acid according to claim 1.

According to a preferred aspect of the present invention, the medium-chain fatty acid is a saturated fatty acid having 8 and/or 10 carbon atoms in the oil or fat composition of the first or second aspect.

According to a further aspect of the present invention, a food or drink contains the oil or fat composition of claims 1 or 2.

### Effects of the Invention

Since the oil or fat composition of the present invention has an action of promoting insulin secretion and an action of improving insulin resistance, it is effective in the treatment of diabetes accompanied by insulin secretion failure that causes a decrease in the amount of insulin secretion, and insulin resistance that causes an impairment of an action of insulin. In addition, since the oil or fat composition of the present invention includes triacylglycerol as an active ingredient, it is highly safe, and can be ingested on a continuous basis reliably due to the absence of concern over side effects.

### BRIEF DESCRIPTION OF THE DRAWINGS

Fig. 1 shows a drawing illustrating results of determination of plasma insulin level in MLCT administration trials.
Fig. 2 shows a drawing illustrating results of determination of plasma insulin level in an MCT administration trial.
Fig. 3 shows a drawing illustrating results of determination of plasma insulin level in an MCT + LCT administration trial.

### PREFERRED MODE FOR CARRYING OUT THE INVENTION

Hereinafter, specific embodiments of the present invention are explained in detail, but the present invention is not in any way limited to the following embodiments.

The oil or fat composition of the present disclosure is used for the treatment of diabetes, to improve insulin resistance and includes triacylglycerol as an active ingredient. In addition, the triacylglycerol added as an active ingredient of the oil or fat composition of the present invention has one or two medium-chain fatty acid residues per molecule, and including as constituent fatty acids a C₆₋₁₀ medium-chain fatty acid and a C₁₆₋₂₄ long-chain fatty acid. More specifically, the triacylglycerol can be in the form of: a medium-chain fatty acid triacylglycerol (hereinafter, referred to as MCT) that includes only a medium-chain fatty acid as a constituent fatty acid per molecule of the triacylglycerol; a long-chain fatty acid triacylglycerol (hereinafter, referred to as LCT) that includes only a long-chain fatty acid; and a medium-chain and long-chain fatty acid triacylglycerol (hereinafter, referred to as M·LCT) that includes a mixture of a medium-chain fatty acid and a long-chain fatty acid. However, the oil or fat composition of the present invention is characterized by including as an active ingredient M·LCT that includes a C₆₋₁₀ medium-chain fatty acid and a C₁₆₋₂₄ long-chain fatty acid per molecule of the triacylglycerol, as constituent fatty acids.

The oil or fat composition of the present invention may also include the MCT and/or LCT in addition to the M·LCT as an active ingredient. It is to be noted that such a triacylglycerol is herein referred to as a medium-chain and long-chain fatty acid triacylglycerol mixture (hereinafter, referred to as MLCT), and is expressed distinctively from M·LCT of triacylglycerol having one or two medium-chain fatty acid residues per molecule, and including a mixture of a medium-chain fatty acid and a long-chain fatty acid as constituent fatty acids.

The triacylglycerol as the active ingredient includes a C₆₋₁₀ medium-chain fatty acid and a C₁₆₋₂₄ long-chain fatty acid, and preferably consists of a C₆₋₁₀ medium-chain fatty acid and a C₁₆₋₂₄ long-chain fatty acid. Examples of the C₆₋₁₀ medium-chain fatty acid include n-hexanoic acid, n-heptanoic acid, n-octanoic acid, n-nonanoic acid, and n-decanoic acid. The C₆₋₁₀ medium-chain fatty acid is preferably a saturated fatty acid having an even number of carbon atoms, and more preferably a saturated fatty acid having 8 and/or 10 carbon atoms (i.e., n-octanoic acid and/or n-decanoic acid). The medium-chain fatty acid can be obtained by hydrolyzing coconut oil, palm kernel oil or the like.

Examples of the C₁₆₋₂₄ long-chain fatty acid include long chain saturated fatty acids such as palmitic acid, stearic acid, arachidic acid, behenic acid and lignoceric acid, long chain unsaturated fatty acids such as palmitoleic acid, oleic acid, linoleic acid, linolenic acid, γ-linolenic acid, arachidonic acid and icosapentaenoic acid. The long-chain fatty acid can be obtained by hydrolyzing soybean oil, rapeseed oil, corn oil, rice bran oil, sesame oil, cotton seed oil, sunflower seed oil, safflower oil, linseed oil, perilla oil, olive oil or the like produced by pressing and purifying a raw oil material for compression.

The triacylglycerol added as an active ingredient of the oil or fat composition of the present invention contains the medium-chain fatty acid and the long-chain fatty acid as constituent fatty acids, but the binding site of these fatty acids is not particularly limited.

The method for producing a triacylglycerol is not particularly limited, and for example, a conventionally well-known transesterification reaction or esterification reaction may be exemplified. The transesterification reaction may include, for example, a chemical transesterification reaction carried out using an inorganic catalyst such as sodium methoxide, and an enzymatic transesterification reaction carried out using lipase or the like. In the present invention, the transesterification reaction may be either chemical or enzymatic. In addition, the transesterification reaction is not particularly limited, which may be either a selective transesterification reaction or a nonselective transesterification reaction.

The esterification reaction may include, for example, an esterification reaction carried out using glycerin and a fatty acid, an esterification reaction carried out using glycerin and a fatty acid ester, an esterification reaction carried out using glycerin and an oil or fat, and the like. In the present invention, esterification reaction may be any of these reactions.

In the oil or fat composition of the present invention, the proportion of the medium-chain fatty acid in the entire constituent fatty acids of the triacylglycerol added as an active ingredient is preferably 15 to 40% by mass, and more preferably 20 to 35% by mass. When the proportion falls within this range, the action of promoting insulin secretion, and the action of improving insulin resistance can be more effectively exhibited. It is to be noted that as a method for determining the proportion of the medium-chain fatty acid occupying the constituent fatty acids, for example, a method including: isolating the triacylglycerol from the oil or fat composition of the present invention by molecular distillation, column chromatography or the like; thereafter subjecting the constituent fatty acids to methyl esterification; and analyzing the product by gas chromatography.

A well-known emulsifying agent may be added to the oil or fat composition of the present invention within the range not to impair the effects of the present invention, for the purpose of further improving storage stability, and for additionally improving cooking aptitude. Specifically, the emulsifying agent may be exemplified by a synthetic emulsifying agent such as a polyglycerin fatty acid ester, a sucrose fatty acid ester, a sorbitan fatty acid ester, polysorbate, a condensed ricinolein fatty acid ester or a monoglycerin fatty acid ester, a natural emulsifying agent such as soybean lecithin, egg yolk lecithin, soybean lysolecithin, egg yolk lysolecithin, enzyme-treated egg yolk, saponin, a plant sterol or milk fat globule membrane.

The oil or fat composition of the present invention effectively acts on animals including human suffering from diabetes who necessitate an increase in the amount of insulin secretion, or potential patients thereof, as well as animals including human suffering from diabetes who necessitate improvement of insulin resistance, or potential patients thereof. In particular, the oil or fat composition of the present disclosure is suitable for the treatment of type II diabetes accompanied by insulin secretion failure that causes a decrease in the amount of insulin secretion, and/or insulin resistance that causes an impairment of an action of insulin. As referred to herein, the prevention means, for example, suppression, delay and the like of the onset, whereas the treatment means, for example, delay of progress, remission, alleviation and amelioration of symptoms, and the like.

The triacylglycerol and the medium-chain fatty acid and long-chain fatty acid that are the constituent fatty acid thereof included in the oil or fat composition of the present invention as active ingredients are broadly present in nature, and also included in natural products which may be edible. Therefore, the oil or fat composition of the present invention is highly safe, and can be suitably used as a medical drug (including those for use in animals), food, animal feed, and the like.

Since it is necessary to perform a pharmacological therapy for diabetes on a continuous basis for a long period of time, ingredients used in medical drugs are required to be accompanied by no concern over side effects even if dosed on a continuous basis. Since the oil or fat composition of the present invention contains triacylglycerol that is highly safe, and accompanied by no concern over side effects as an active ingredient, it can be reliably dosed on a continuous basis also when used in medical drugs. In addition, since dosing of the oil or fat composition of the present invention can be easily controlled, the composition is suited for pharmacological therapies including alimentary therapy in combination with exercise therapy carried out in the treatment of type II diabetes.

The administration route of the oil or fat composition of the present invention when used as a medical drug is preferably an oral administration route since the majority of the triacylglycerol added as an active ingredient of the oil or fat composition of the present invention is absorbed into a living body through the mucosa of intestinal tract (small intestine). Examples of the formulation suitable for the oral administration include capsules, tablets, pills, powders, subtle granules, granules, liquids and solutions, syrups, and the like. The oil or fat composition of the present invention is preferably prepared into a formulation of a medical drug composition containing a triacylglycerol added as an active ingredient, and a pharmacologically and pharmaceutically acceptable additive. Examples of the pharmacologically and pharmaceutically acceptable additive include excipients such as glucose, lactose, crystalline cellulose and starch, disintegrants, binding agents, coating agents, coloring matters, diluents and the like, and a substance that is commonly used in formulation arts in general and does not react with the triacylglycerol added as an active ingredient may be used.

Since the oil or fat composition of the present invention is highly safe, and is not accompanied by concern over side effects continuous dosing as described above, the dosage amount of a preexisting drug is lowered by use in combination, whereby side effect of the same can be diminished. When used in combination with any other drug, the composition and the other drug may be included in a single pharmaceutical composition like a combination drug, or may be included in distinct pharmaceutical compositions.

The amount of administration of the medical drug prepared using the oil or fat composition of the present invention may be appropriately determined depending on a variety of conditions such as symptoms, age and body weight of the patient, the administration method, the administration duration, and the type of the treatment which may be prophylactic or therapeutic. For example, in the case in which therapies of diabetes of human (adult: 60 kg) are intended, the effective amount is usually 200 to 1000 mg/kg of body weight/ day in terms of the triacylglycerol added as an active ingredient and this dose may be administered once or divided for administration over several times, with appropriate adjustment that may be made so as to fall within the above range depending on the administration timing and the like.

The oil or fat composition of the present invention can be ingested as, for example, foods such as health foods and nutritional supplementary foods by packing into a soft capsule and/or processing. Also, the oil or fat composition of the present invention may be either directly ingested as is or after being processed into a powdery lipid or a liquidified emulsion lipid, or indirectly ingested by further processing these for use in general foods.

General foods in which the oil or fat composition of the present invention can be used are not particularly limit as long as they are processed food in which a lipid is used, and for example, breads and confectioneries such as breads, cakes, cookies, biscuits, doughnuts, muffins, scones, chocolates, gummi candies, snack foods, whipped creams and ice creams; drinks such as juice drinks, nutrition drinks and sports drinks; processed and seasoned foods such as soups, dressings, sources, mayonnaises, butters, margarines and prepared margarines; fat spreads, shortenings, bakery mixes, sautéing oils, frying oils, fried foods, processed meat products, frozen foods, noodles, retort foods, liquid diets, dysphagia diets, and the like may be exemplified.

In the case in which the oil or fat composition of the present invention is used for producing a food or drink ingested by diabetic patients or potential patients thereof, for example, when prepared for adults (body weight: 60 kg), taking into consideration the type of the food prepared, other ingredients included in the food to be ingested, and the like, it may be prepared such that the amount of ingested triacylglycerol, an active ingredient, becomes 200 to 1000 mg/kg of body weight/ day, which may be appropriately adjusted to fall within this range depending on the timing and the like of the ingestion.

### EXAMPLES

Hereinafter, the present invention will be explained in more detail by way of Examples, but the present invention is not in any way limited thereto.

### [Test Example 1] Study of Effect of Promoting Insulin Secretion (1)

Using rats subjected to carbohydrate load, an effect of promoting plasma insulin secretion by the oil or fat composition of the present invention (MLCT) was studied with LCT as a control.

### <Production Example 1> Method for Producing Sample 1

To 10 ml of a 40% glucose (D(+)-Glucose, manufactured by Wako Pure Chemical Industries, Ltd.) solution were added 2 g of LCT (trade name: Nisshin Canola Oil, manufactured by Nisshin OilliO Group, Ltd.) and 200 mg of bovine serum albumin (manufactured by SIGMA Co.), and the solution obtained was subjected to an ultrasonic treatment (5 min x 2, for 10 min in total) while cooling on ice to obtain Sample 1. Afatty acid composition of LCT (trade name: Nisshin Canola Oil, manufactured by Nisshin OilliO Group, Ltd.) used as a control is shown in Table 1.

**[Table 1]**

| table 1 | |
|---|---|
| Fatty acid composition | Proportion (% by mass) |
| C16:0 | 4.4 |
| C18:0 | 2.1 |
| C18:1 | 59.4 |
| C18:2 | 20.7 |
| C18:3 | 10.7 |
| Other long-chain fatty acid (C20∼C24) | 2.7 |

### <Production Example 2> Method for Producing Sample 2

MCT (trade name: O.D.O, constituent fatty acids: n-octanoic acid/ n-decanoic acid = 3/1, manufactured by Nisshin OilliO Group, Ltd.), and LCT (trade name: Nisshin Canola Oil, manufactured by Nisshin OilliO Group, Ltd.) were mixed at a ratio of 14:86 (mass ratio) to give an oil mixture. To the oil mixture obtained was added a lipase powder in an amount of 0.1% by mass of with respect to the oil mixture, and thereafter the mixture was stirred at 60°C for 15 hrs to allow for a transesterification reaction. Next, the lipase powder was filtered off from the reaction product, and the filtrate was washed with water and dried, followed by decolorization and deodorization to obtain a transesterified oil of MCT with LCT (MLCT (1)). Then, to 10 ml of a 40% glucose (D(+)-Glucose, manufactured by Wako Pure Chemical Industries, Ltd.) solution were added 2 g of the aforementioned MLCT (1) and 200 mg of bovine serum albumin (manufactured by SIGMA Co.), and the solution obtained was subjected to an ultrasonic treatment (5 min x 2, for 10 min in total) while cooling on ice to obtain Sample 2.

A triacylglycerol composition and a fatty acid composition of MLCT obtained in Production Example 2 are shown in Table 2 and Table 3, respectively. It is to be noted that the triacylglycerol composition was determined by gas chromatography using Silicone GS-1 manufactured by GL Sciences, Inc. as a column, and the fatty acid composition was determined in accordance with "Standard fat and oil analytical test methods (1996)".

**[Table 2]**

| table 2 | |
|---|---|
| Triacylglycerol composition | Proportion (% by mass) |
| 3M0L | 0.5 |
| 2M1L | 10.1 |
| 1M2L | 36.2 |
| 0M3L | 53.2 |
| Total | 100 |

| | |
|---|---|
| 3M0L: including 3 medium-chain fatty acid residues and 0 long-chain fatty acid residues per molecule 2M1L: including 2 medium-chain fatty acid residues and 1 long-chain fatty acid residue per molecule 1M2L: including 1 medium-chain fatty acid residue and 2 long-chain fatty acid residues per molecule 0M3L: including 0 medium-chain fatty acid residues and 3 long-chain fatty acid residues per molecule | |

**[Table 3]**

| table 3 | |
|---|---|
| Fatty acid composition | Proportion (% by mass) |
| C8:0 | 8.8 |
| C10:0 | 3.1 |
| C16:0 | 2.9 |
| C18:0 | 1.2 |
| C18:1 | 57.0 |
| C18:2 | 17.7 |
| C18:3 | 7.2 |
| Other long-chain fatty acid (C20∼C24) | 2.1 |

For the test, 7-weeks old male SD rats (purchased from Japan SLC, Inc.) were used. After the rats (19 animals) were kept for acclimation for one week, a carbohydrate tolerance test was conducted by a crossover study in which Sample 1 and Sample 2 were administered on the 8th week and 9th week. More specifically, in order to enable comparison of an effect achieved by MLCT (1) in an identical individual, a crossover study was employed in which the first carbohydrate tolerance test was followed by a wash-out period for one week, and then the second carbohydrate tolerance test was carried out. During the feeding period, the rats were kept separately in each stainless mesh cage in an environment involving a temperature of 23±1°C and a humidity of 50±10%, 12 hrs under a light-dark cycle (light: 8:00 to 20:00 h). Free intake of water and a solid animal feed (trade name: PicoLab Rodent Diet 20 5053, manufactured by Japan SLC, Inc.) was permitted. On the day before starting the test, the animals were starved from 17:00, and on the first day of the test, administration was started from 10:00.

The test was performed with two trials including "LCT administration trial (Sample 1 administration trial)" in which LCT was administered concomitantly with carbohydrate loading, and "MLCT (1) administration trial (Sample 2 administration trial) in which MLCT (1) was administered concomitantly with carbohydrate load. Samples 1 and 2 were orally administered to the rats using a probe. The amount of the administration was 6 µl per gram of body weight of the rat. It is to be noted that the final amounts of the administration were 2 g and 1 g of glucide and lipid, respectively, per kg of body weight of the rat.

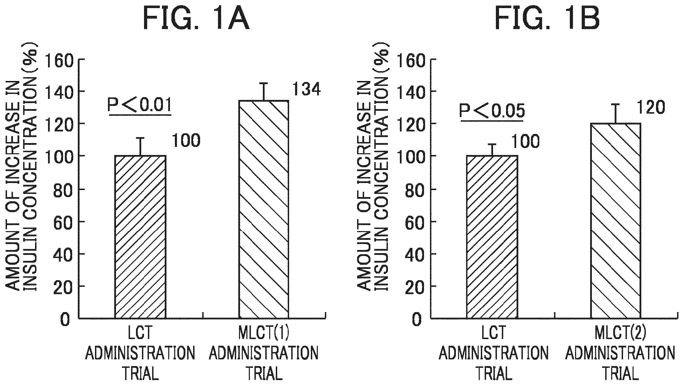

Before and 20 min after the administration, the blood was collected via the tail vein, and the plasma insulin level (ng/ml) was determined. It is to be noted that the plasma insulin level was determined according to an ELISA method using a commercially available kit (trade name: Rat Insulin ELISA kit, manufactured by Mercodia AB). The test results are shown in Fig. 1 (A). The amount of an increase in the plasma insulin level in the MLCT (1) administration trial was defined in terms of a relative value (%), when an average value of the amount of an increase in the plasma insulin level in the LCT administration trial was assumed to be 100, and represented by the average value ± standard error of the relative value. For a test of the significant difference between the trials, a Student t-test was employed, and the presence of the significant difference was decided with a significance level of less than 5%.

As shown in Fig. 1 (A), the amount of an increase in the plasma insulin level of the MLCT (1) administration trial exhibited a significantly (p < 0.01) high value as compared with the LCT administration trial. This revealed that the transesterified oil of MCT with LCT (MLCT) promoted the secretion of insulin. It is to be noted that the average value (absolute value) of the amount of an increase in the plasma insulin level of the LCT administration trial was 0.86 ± 0.10 ng/ml, and the average value (absolute value) of the amount of an increase in the plasma insulin level of the MLCT (1) administration trial was 1.15 ± 0.10 ng/ml.

### [Test Example 2] Study of Effect of Promoting Insulin Secretion (2)

Using rats subjected to carbohydrate load, an effect of promoting plasma insulin secretion by the oil or fat composition of the present invention (MLCT) having a triacylglycerol composition that is different from Test Example 1 was studied with LCT as a control.

### <Production Example 3>Method for Producing Sample 3

MCT (trade name: Scholey 64G, constituent fatty acids: n-octanoic acid/n-decanoic acid = 3/2, manufactured by Nisshin OilliO Group, Ltd.), and LCT (trade name: Nisshin Corn Oil, manufactured by Nisshin OilliO Group, Ltd.) were mixed at a ratio of 30:70 (mass ratio) to give an oil mixture. The oil mixture obtained was subjected to a transesterification reaction, decolorization treatment, and deodorization treatment in a similar manner to Production Example 2, to obtain a transesterified oil of MCT with LCT (MLCT (2)). Then, to 10 ml of a 40% glucose (D(+)-Glucose, manufactured by Wako Pure Chemical Industries, Ltd.) solution were added 2 g of the aforementioned MLCT (2) and 200 mg of bovine serum albumin (manufactured by SIGMA Co.), and the solution obtained was subjected to an ultrasonic treatment (5 min x 2, for 10 min in total) while cooling on ice to obtain Sample 3.

A triacylglycerol composition and a fatty acid composition of MLCT obtained in Production Example 3 are shown in Table 4 and Table 5, respectively. The determination was carried out similarly to the method described above.

**[Table 4]**

| table 4 | |
|---|---|
| Triacylglycerol composition | Proportion (% by mass) |
| 3M0L | 5.5 |
| 2M1L | 27.3 |
| 1M2L | 44.3 |
| 0M3L | 22.9 |
| Total | 100 |

| | |
|---|---|
| 3M0L: including 3 medium-chain fatty acid residues and 0 long-chain fatty acid residues per molecule 2M1L: including 2 medium-chain fatty acid residues and 1 long-chain fatty acid residue per molecule 1M2L: including 1 medium-chain fatty acid residue and 2 long-chain fatty acid residues per molecule 0M3L: including 0 medium-chain fatty acid residues and 3 long-chain fatty acid residues per molecule | |

**[Table 5]**

| table 5 | |
|---|---|
| Fatty acid composition | Proportion (% by mass) |
| C8:0 | 22.5 |
| C10:0 | 12.7 |
| C16:0 | 6.8 |
| C18:0 | 1.2 |
| C18:1 | 19.1 |
| C18:2 | 36.9 |
| C18:3 | 0.6 |
| Other long-chain fatty acid (C20∼C24) | 0.2 |

For the test, 7-weeks old male SD rats (purchased from Japan SLC, Inc.) were used. After the rats (19 animals) were kept for acclimation for one week, a carbohydrate tolerance test was conducted by a crossover study in which Sample 1 and Sample 3 were administered on the 8th week and 9th week. The feeding conditions were similar to Test Example 1.

The test was performed with two trials including "LCT administration trial (Sample 1 administration trial)" in which LCT was administered concomitantly with carbohydrate loading, and "MLCT (2) administration trial (Sample 3 administration trial) in which MLCT (2) was administered concomitantly with carbohydrate load. Samples 1 and 3 were orally administered to the rats using a probe. The amount of the administration was 6 µl per gram of body weight of the rat. It is to be noted that the final amounts of the administration were 2 g and 1 g of glucide and lipid, respectively, per kg of body weight of the rat.

Before and 20 min after the administration, the blood was collected via the tail vein, and the plasma insulin level (ng/ml) was determined according to a similar method to Test Example 1. The test results are shown in Fig. 1 (B). The amount of an increase in the plasma insulin level in the MLCT (2) administration trial was defined in terms of a relative value (%), when an average value of the amount of an increase in the plasma insulin level in the LCT administration trial was assumed to be 100, and represented by the average value ± standard error of the relative value. For a test of the significant difference between the trials, a Student t-test was employed, and the presence of the significant difference was decided with a significance level of less than 5%.

As shown in Fig. 1 (B), the amount of an increase in the plasma insulin level of the MLCT (2) administration trial exhibited a significantly (p < 0.05) high value as compared with the LCT administration trial. This also revealed that the transesterified oil of MCT with LCT (MLCT) promoted the secretion of insulin. It is to be noted that the average value (absolute value) of the amount of an increase in the plasma insulin level of the LCT administration trial was 1.25 ± 0.10 ng/ml, and the average value (absolute value) of the amount of an increase in the plasma insulin level of the MLCT (2) administration trial was 1.50 ± 0.14 ng/ml.

### [Test Example 3] Study of Effect of Promoting Insulin Secretion (3)

Using rats subjected to carbohydrate load, an effect of promoting plasma insulin secretion by MCT was studied with LCT as a control.

### <Production Example 4> Method for Producing Sample 4

To 10 ml of a 40% glucose (D(+)-Glucose, manufactured by Wako Pure Chemical Industries, Ltd.) solution were added 2 g of MCT (trade name: O.D.O, constituent fatty acids: n-octanoic acid/ n-decanoic acid = 3/1, manufactured by Nisshin OilliO Group, Ltd.) and 200 mg of bovine serum albumin (manufactured by SIGMA Co.), and the solution obtained was subjected to an ultrasonic treatment (5 min x 2, for 10 min in total) while cooling on ice to obtain Sample 4.

For the test, 7-weeks old male SD rats (purchased from Japan SLC, Inc.) were used. After the rats (18 animals) were kept for acclimation for one week, a carbohydrate tolerance test was conducted by a crossover study in which Sample 1 and Sample 4 were administered on the 8th week and 9th week. The feeding conditions were similar to Test Example 1.

The test was performed with two trials including "LCT administration trial (Sample 1 administration trial)" in which LCT was administered concomitantly with carbohydrate loading, and "MCT administration trial (Sample 4 administration trial) in which MCT was administered concomitantly with carbohydrate load. Samples 1 and 4 were orally administered to the rats using a probe. The amount of the administration was 6 µl per gram of body weight of the rat. It is to be noted that the final amounts of the administration were 2 g and 1 g of glucide and lipid, respectively, per kg of body weight of the rat.

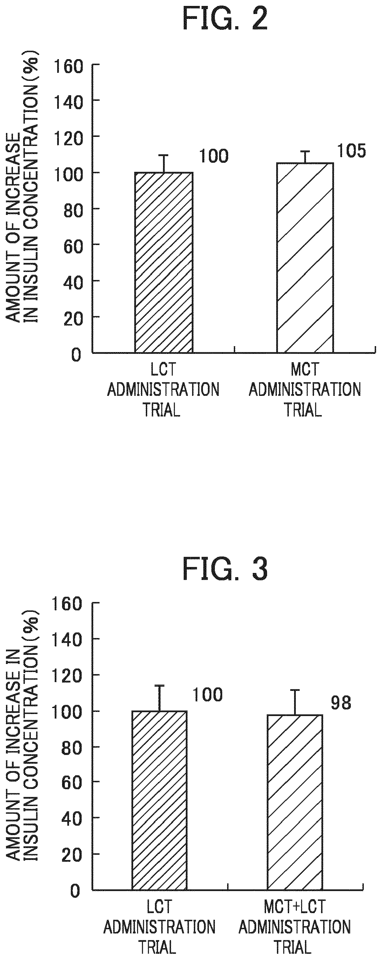

Before and 20 min after the administration, the blood was collected via the tail vein, and the plasma insulin level (ng/ml) was determined according to a similar method to Test Example 1. The test results are shown in Fig. 2. The amount of an increase in the plasma insulin level in the MCT administration trial was defined in terms of a relative value (%), when an average value of the amount of an increase in the plasma insulin level in the LCT administration trial was assumed to be 100, and represented by the average value ± standard error of the relative value. For a test of the significant difference between the trials, a Student t-test was employed, and the presence of the significant difference was decided with a significance level of less than 5%.

As shown in Fig. 2, a significant difference was not found between the amount of an increase in the plasma insulin level of the MCT administration trial, and the amount of an increase in the plasma insulin level of the LCT administration trial. It is to be noted that the average value (absolute value) of the amount of an increase in the plasma insulin level of the LCT administration trial was 1.27 ± 0.12 ng/ml, and the average value (absolute value) of the amount of an increase in the plasma insulin level of the MCT administration trial was 1.34 ± 0.08 ng/ml.

### [Test Example 4] Study of Effect of Promoting Insulin Secretion (4)

Using rats subjected to carbohydrate load, an effect of promoting plasma insulin secretion by an oil mixture of MCT and LCT (MCT + LCT) was studied with LCT as a control.

### <Production Example 5> Method for Producing Sample 5

MCT (trade name: O.D.O, constituent fatty acids: n-octanoic acid/n-decanoic acid = 3/1, manufactured by Nisshin OilliO Group, Ltd.) and LCT (trade name: Nisshin Canola Oil, manufactured by Nisshin OilliO Group, Ltd.) were mixed at a ratio of 14:86 (mass ratio) to give an oil mixture of MCT and LCT (MCT + LCT). Then, to 10 ml of a 40% glucose (D(+)-Glucose, manufactured by Wako Pure Chemical Industries, Ltd.) solution were added 2 g of the oil mixture, and 200 mg of bovine serum albumin (manufactured by SIGMA Co.), and the solution obtained was subjected to an ultrasonic treatment (5 min x 2, for 10 min in total) while cooling on ice to obtain Sample 5.

For the test, 7-weeks old male SD rats (purchased from Japan SLC, Inc.) were used. After the rats (14 animals) were kept for acclimation for one week, a carbohydrate tolerance test was conducted by a crossover study in which Sample 1 and Sample 5 were administered on the 8th week and 9th week. The feeding conditions were similar to Test Example 1.

The test was performed with two trials including "LCT administration trial (Sample 1 administration trial)" in which LCT was administered concomitantly with carbohydrate loading, and "MCT + LCT administration trial (Sample 5 administration trial) in which the oil mixture of MCT and LCT was administered concomitantly with carbohydrate load. Samples 1 and 5 were orally administered to the rats using a probe. The amount of the administration was 6 µl per gram of body weight of the rat. It is to be noted that the final amounts of the administration were 2 g and 1 g of glucide and lipid, respectively, per kg of body weight of the rat.

Before and 20 min after the administration, the blood was collected via the tail vein, and the plasma insulin level (ng/ml) was determined according to a similar method to Test Example 1. The test results are shown in Fig. 3. The amount of an increase in the plasma insulin level in the MCT + LCT administration trial was defined in terms of a relative value (%), when an average value of the amount of an increase in the plasma insulin level in the LCT administration trial was assumed to be 100, and represented by the average value ± standard error of the relative value. For a test of the significant difference between the trials, a Student t-test was employed, and the presence of the significant difference was decided with a significance level of less than 5%.

As shown in Fig. 3, a significant difference was not found between the amount of an increase in the plasma insulin level of the MCT + LCT administration trial, and the amount of an increase in the plasma insulin level of the LCT administration trial. It is to be noted that the average value (absolute value) of the amount of an increase in the plasma insulin level of the LCT administration trial was 1.28 ± 0.18 ng/ml, and the average value (absolute value) of the amount of an increase in the plasma insulin level of the MCT + LCT administration trial was 1.25 ± 0.18 ng/ml.

From the results of Test Examples 1 to 4 in the foregoing, the effect of promoting plasma insulin secretion found in the case of the transesterified oil of MCT with LCT (MLCT) was reveled to result from the triacylglycerol having one or two medium-chain fatty acid residues per molecule among the triacylglycerol included in MLCT.

### [Test Example 5] Study of Effect of Improving Insulin Resistance

Using model animals in which insulin resistance was developed by way of feeding of a high fat diet for a long period of time, an effect of improving insulin resistance of the oil or fat composition of the present invention (MLCT) was studied with LCT as a control.

### <Production Example 6> Method for Producing Oil or Fat composition

MCT (trade name: O.D.O, constituent fatty acids: n-octanoic acid/n-decanoic acid = 3/1, manufactured by Nisshin OilliO Group, Ltd.), and LCT (trade name: Nisshin Canola Oil, manufactured by Nisshin OilliO Group, Ltd.) were mixed at a ratio of 14:86 (mass ratio) to give an oil mixture. To the oil mixture obtained was added a lipase powder in an amount of 0.1% by mass of with respect to the oil mixture, and thereafter the mixture was stirred at 60°C for 15 hrs to allow for a transesterification reaction. Next, the lipase powder was filtered off from the reaction product, and the filtrate was washed with water and dried, followed by decolorization and deodorization to obtain a transesterified oil of MCT with LCT (MLCT) that is the oil or fat composition of the present invention.

A triacylglycerol composition and a fatty acid composition of MLCT obtained in Production Example 6 are shown in Table 6 and Table 7, respectively. The determination was carried out similarly to the method described above.

**[Table 6]**

| table 6 | |
|---|---|
| Triacylglycerol composition | Proportion (% by mass) |
| 3M0L | 0.5 |
| 2M1L | 9.5 |
| 1M2L | 34.7 |
| 0M3L | 55.3 |
| total | 100 |

| | |
|---|---|
| 3H0L: including 3 medium-chain fatty acid residues and 0 long-chain fatty acid residues per molecule 2M1L: including 2 medium-chain fatty acid residues and 1 long-chain fatty acid residue per molecule 1H2L: including 1 medium-chain fatty acid residue and 2 long-chain fatty acid residues per molecule 0M3L: including 0 medium-chain fatty acid residues and 3 long-chain fatty acid residues per molecule | |

**[Table 7]**

| table 7 | |
|---|---|
| Fatty acid composition | Proportion (% by mass) |
| C8:0 | 8.3 |
| C10:0 | 2.7 |
| C16:0 | 2.8 |
| C18:0 | 1.2 |
| C18:1 | 57.5 |
| C18:2 | 18.1 |
| C18:3 | 7.2 |
| Other long-chain fatty acid (C20∼C24) | 2.2 |

### <Production Example 7> Method for Producing Test Animal Feed

As a test animal feed, in accordance with a formulation of AIN93 standard diet, an animal feed designed using LCT (trade name: Nisshin Canola Oil, manufactured by Nisshin OilliO Group, Ltd.) or MLCT obtained in Production Example 6 was used as the lipid. A composition of the test animal feed is shown in Table 8. First, each ingredient other than the lipid (LCT or MLCT) was mixed, and thereafter the lipid was mixed followed by homogenization to obtain a test animal feed (LCT diet or MLCT diet).

**[Table 8]**

| table 8 | | |
|---|---|---|
| Ingredient | LCT diet (% by mass) | MLCT diet (% by mass) |
| Corn starch | 45.7486 | 45.7486 |
| Milk casein | 20 | 20 |
| Gelatinized corn starch | 13.2 | 13.2 |
| Granulated sugar | 4 | 4 |
| MLCT | 0 | 7 |
| LCT | 7 | 0 |
| Cellulose powder | 5 | 5 |
| Mineral mix | 3. 5 | 3.5 |
| Vitamin mix | 1 | 1 |
| L-cystine | 0.3 | 0.3 |
| Choline bitartrate | 0.25 | 0.25 |
| tert-butylhydroquinone | 0.0014 | 0.0014 |

For the test, insulin resistant model rats (Reference Document: Han et al, Diabetes, 46, p1761 - p1767, 1997) were used. More specifically, male Sprague-Dawley rats (purchased from Japan SLC, Inc.) were allowed to ingest a high-fat animal feed having a composition shown in Table 9 until 4 weeks old to 12 weeks old to develop insulin resistance, and thus these rats were used. During the feeding period, the rats were kept separately in each stainless mesh cage in a environment involving a temperature of 23 ± 1°C and a humidity of 50 ± 10%, under a 12/12-hour light-dark cycle (light: 8:00 to 20:00 h). Free intake of water and the high fat animal feed was permitted.

**[Table 9]**

| table 9 | |
|---|---|
| granulated sugar | 34.7286 |
| Casein | 29.34 |
| Lard | 18 |
| Canola oil | 10 |
| Methionine | 0.5 |
| Mineral mix | 5.1 |
| Vitamin mix | 2.2 |
| Choline bitartrate | 0.13 |
| tert-butylhydroquinone | 0.0014 |

| | |
|---|---|
| *: trade name Nisshin Canola Oil, manufactured by Nisshin OilliO Group, Ltd. | |

Thirty insulin resistant model rats described above were divided into two groups (each 15 animals) such that the body weight, the blood glucose level, and the plasma insulin level became equivalent, and the groups were designated as an "LCT diet fed group (control group)" in which the LCT diet was ingested, and an "MLCT diet fed group" in which the MLCT diet was ingested, respectively. It is to be noted that Glucose C2 Test Wako (manufactured by Wako Pure Chemical Industries, Ltd.) was used for the measurement of the blood glucose level, and Rat Insulin ELISA kit (manufactured by Mercodia AB) was used for the measurement of the insulin concentration to analyze the tail vein blood of each rat.

After thus grouping, the rats were kept separately in each stainless mesh cage in a environment involving a temperature of 23 ± 1°C and a humidity of 50 ± 10%, under a 12/12-hour light-dark cycle (light: 8:00 to 20:00 h). Free intake of water and the test animal feed was permitted.

In this study, in order to verify the effect of improving the insulin resistance, the insulin resistant model rats were kept for 6 weeks while allowing for ingestion of the test animal feed, and thereafter an oral carbohydrate tolerance test was carried out to analyze the blood glucose level after the carbohydrate loading and time dependent alteration of the plasma insulin level. More specifically, the rats which had been starved overnight (for 16 hrs) were orally administered with glucose in an amount of 1.5 g per kg of the body weight. The blood was collected via their tail vein before the administration, as well as after 30 min, 60 min and 120 min of the administration, and the blood glucose level and the insulin concentration were determined. It is to be noted that Glucose C2 Test Wako (manufactured by Wako Pure Chemical Industries, Ltd.) was used for the measurement of the blood glucose level, and Rat Insulin ELISA kit (manufactured by Mercodia AB) was used for the measurement of the insulin concentration. From curve graphs produced by means of the measurement values to show the time dependent alteration of the blood glucose level and the insulin concentration, the areas under the curve (Area Under the Curve: AUC) were calculated, and the resulting values was defined to be amounts of change of the blood glucose level and plasma insulin level after the carbohydrate load, respectively. Then, with respect to the amounts of change in each group, the average value and the standard error were derived, and a Student t-test was employed to carry out a significance test between the groups. Thus, the presence of the significant difference was decided with a significance level of less than 5%. The results are shown in Table 10.

**[Table 10]**

| table 10 | | |
|---|---|---|
| | LCT diet fed group | MLCT diet fed group |
| Blood glucose level AUC (mg/dL·min) | 4242±382 | 4244±234 |
| Insulin concentration AUC ( *µ*g/L·min) | 215±22 | 117±21** |

| | | |
|---|---|---|
| ** statistically significant difference from LCT diet fed group present (p < 0.01) | | |

As shown in Table 10, no significant difference of the blood glucose level AUC was found between the LCT diet fed group and the MLCT diet fed group; however, a significantly low value of the insulin concentration AUC was exhibited in the MLCT diet fed group as compared with the LCT diet fed group. In other words, in the MLCT diet fed group, a blood glucose level which was the same as the level in the LCT diet fed group was maintained, although insulin secretion was less than that in the LCT diet fed group, and thus improvement of insulin resistance as a result of ingestion of MLCT was observed.

One cause of development of insulin resistance is considered to be a failure in appropriate secretion of adiponectin having an action of enhancing insulin sensitivity, from fat cells, due to obesity, particularly accumulation of visceral fat (see Kadowaki et al, J Clin Invest, 116, p1784-p1792, 2006). Thus, alteration of the amount of adiponectin secretion, and the amount of visceral fat accompanied by improvement of insulin resistance in rats were examined.

Measurement of the adiponectin concentration in plasma was conducted on the rats described above which had been allowed to ingest the test animal feed for 6 weeks feed, before performing the oral carbohydrate tolerance test. More specifically, the blood was collected via the tail vein just before administration of glucose to the rat, and the obtained blood was separated by centrifugation at 3,000 rpm for 10 min to fractionate the supernatant plasma. The adiponectin concentration in plasma was determined using an ELISA method (mouse /rat adiponectin ELISA kit, manufactured by Otsuka Pharmaceutical Co., Ltd.). Then, with respect to the measurements in each group, the average value and the standard error were derived, and a Student t-test was employed to carry out a significance test between the groups. Thus, the presence of the significant difference was decided with a significance level of less than 5%. The results are shown in Table 11.

**[Table 11]**

| table 11 | | |
|---|---|---|
| | LCT diet fed group | MLCT diet fed group |
| Adiponectin concentration (*µ*g/ml) | 1.87±0.09 | 2.12±0.07* |

| | | |
|---|---|---|
| * statistically significant difference from LCT diet fed group present (p < 0.05) | | |

As shown in Table 11, in the MLCT diet fed group a significantly higher value of the adiponectin concentration in plasma was exhibited as compared with the LCT diet fed group. From this result, ingestion of MLCT leads to amelioration of adiponectin secretion in the MLCT diet fed group, and thus it is believed that the action of enhancing insulin sensitivity by adiponectin improved insulin resistance.

Next, in the rats after the oral carbohydrate tolerance test, the amount of visceral fat, the amount of body weight gain, and the amount of ingested test animal feed were measured. The rats kept for 2 days while allowing for ingestion of the test animal feed after the oral carbohydrate tolerance test were sacrificed by blood removal under anesthesia with diethyl ether. The epididymal fat, the perirenal fat, and the mesenteric fat were extirpated, and each weight was measured to determine the amount of epididymal fat, the amount of perirenal fat, and the amount of mesenteric fat, respectively. In addition, the total amount of fat of the amount of epididymal fat, the amount of perirenal fat, and the amount of mesenteric fat was defined as the amount of visceral fat. The amount of body weight gain was determined by measuring the body weight of each rat when the administration of the test animal feed was started and that of just before the dissection, and calculating their difference. The amount of the ingested test animal feed was determined by measuring the total amount of the test animal feed ingested by each rat during the feeding period, and dividing the total amount by the number of days of the the feeding period to derive in terms of the average amount ingested per day. Then, with respect to the amounts in each group, the average value and the standard error were derived, and a Student t-test was employed to carry out a significance test between the groups. Thus, the presence of the significant difference was decided with a significance level of less than 5%. The results of measurement of the amount of visceral fat are shown in Table 12, and the results of the measurement of the amount of body weight gain and the amount of the ingested test sample are shown in Table 13.

**[Table 12]**

| table 12 | | |
|---|---|---|
| | LCT diet fed group | MLCT diet fed group |
| Amount of epididymal fat(g) | 14.6±0.9 | 14.5±0.7 |
| Amount of perirenal fat(g) | 19.9±1.0 | 20.3±1.0 |
| Amount of mesenteric fat(g) | 11.4±0.6 | 9.6±0.6 * |
| Amount of visceral fat(g) | 45.9±2.3 | 44.5±2.2 |

| | | |
|---|---|---|
| * statistically significant difference from LCT diet fed group present (P < 0.05) | | |

**[Table 13]**

| table 13 | | |
|---|---|---|
| | LCT diet fed group | MLCT diet fed group |
| Body weight when administration of the test animal chaw was started(g) | 459±10 | 459±9 |
| Upon dissection(g) | 515±9 | 514±10 |
| Amount of body weight gain(g) | 56±6 | 55±4 |
| Amount of ingested test animal chaw(g/day) | 19.0±0.4 | 18.4±0.5 |

As shown in Table 12, a significant difference was not found between the amounts of visceral fat in the LCT diet fed group and the MLCT diet fed group; however, the amount of mesenteric fat was significantly lower in the MLCT diet fed group as compared with the LCT diet fed group. Therefore, it is believed that MLCT contributes to amelioration of secretion of adiponectin. It is to be noted that as shown in Table 13, the amount of body weight gain was comparative in the LCT diet fed group and in the MLCT diet fed group, and the differences in the ingestion amount and the growth were not found depending on the difference of the test animal feed.

### INDUSTRIAL APPLICABILITY

The oil or fat composition of the present invention is effective in amelioration of conditions such as insulin secretion failure and insulin resistance found in diabetic patients, and thus can be used in fields of medicine and food for diabetes.

## Claims

1. An oil or fat composition for use in the treatment of diabetes to improve insulin resistance, the composition comprising triacylglycerol as an active ingredient, the triacylglycerol having one or two medium-chain fatty acid residues per molecule, and comprising a C6-10 medium-chain fatty acid and a C16-24 long-chain fatty acid as constituent fatty acids wherein the C16-24 long-chain fatty acid is selected from the group consisting of palmitic acid, stearic acid, arachidic acid, behenic acid, lignoceric acid, palmitoleic acid, oleic acid, linoleic acid, linolenic acid, γ-linolenic acid, arachidonic acid and icosapentaenoic acid,-wherein the constituent fatty acid of the triacylglycerol consists of a C6-10 medium-chain fatty acid and a C16-24 long-chain fatty acid wherein the C16-24 long-chain fatty acid is selected from the group consisting of palmitic acid, stearic acid, arachidic acid, behenic acid, lignoceric acid, palmitoleic acid, oleic acid, linoleic acid, linolenic acid, γ-linolenic acid, arachidonic acid and icosapentaenoic acid.

2. The oil or fat composition for use in the treatment of diabetes to improve insulin resistance according to claim 1, wherein the medium-chain fatty acid is at least one selected from the group consisting of a saturated fatty acid having 8 carbon atoms and a saturated fatty acid having 10 carbon atoms.

3. A food or drink for use in the treatment of diabetes to improve insulin resistance comprising the oil or fat composition according to any one of claims 1 or 2.

## Patentansprüche

1. Öl- oder Fettzusammensetzung zur Verwendung bei der Behandlung von Diabetes zur Verbesserung von Insulinresistenz, wobei die Zusammensetzung Triacylglycerin als einen Wirkstoff umfasst, wobei das Triacylglycerin einen oder zwei mittelkettige Fettsäurereste pro Molekül aufweist, und umfassend eine C6-10-mittelkettige Fettsäure und eine C16-24-langkettige Fettsäure als konstituierende Fettsäuren, wobei die C16-24-langkettige Fettsäure aus der Gruppe gewählt ist, bestehend aus Palmitinsäure, Stearinsäure, Arachinsäure, Behensäure, Lignocerinsäure, Palmitoleinsäure, Ölsäure, Linolsäure, Linolensäure, γ-Linolensäure, Arachidonsäure und Icosapentaensäure, wobei die konstituierende Fettsäure des Triacylglycerins aus einer C6-10-mittelkettigen Fettsäure und einer C16-24-langkettigen Fettsäure besteht, wobei die C16-24-langkettige Fettsäure aus der Gruppe gewählt ist, bestehend aus Palmitinsäure, Stearinsäure, Arachinsäure, Behensäure, Lignocerinsäure, Palmitoleinsäure, Ölsäure, Linolsäure, Linolensäure, γ-Linolensäure, Arachidonsäure und Icosapentaensäure.

2. Öl- oder Fettzusammensetzung zur Verwendung bei der Behandlung von Diabetes zur Verbesserung von Insulinresistenz nach Anspruch 1, wobei die mittelkettige Fettsäure mindestens eine ist, gewählt aus der Gruppe, bestehend aus einer gesättigten Fettsäure mit 8 Kohlenstoffatomen und einer gesättigten Fettsäure mit 10 Kohlenstoffatomen.

3. Nahrungsmittel oder Getränk zur Verwendung bei der Behandlung von Diabetes zur Verbesserung von Insulinresistenz, umfassend die Öl- oder Fettzusammensetzung nach irgendeinem der Ansprüche 1 oder 2.

## Revendications

1. Composition d'huile ou de graisse pour une utilisation dans le traitement du diabète pour améliorer l'insulinorésistance, la composition comprenant du triacylglycérol en tant que substance active, le triacylglycérol comportant un ou deux résidus acide gras à chaîne moyenne par molécule, et comprenant un acide gras à chaîne moyenne en C6-10 et un acide gras à chaîne longue en C16-24 en tant qu'acides gras constitutifs dans laquelle l'acide gras à chaîne longue en C16-24 est choisi dans le groupe constitué de l'acide palmitique, de l'acide stéarique, de l'acide arachidique, de l'acide béhénique, de l'acide lignocérique, de l'acide palmitoléique, de l'acide oléique, de l'acide linoléique, de l'acide linolénique, de l'acide γ-linolénique, de l'acide arachidonique et de l'acide icosapentaénoïque, dans laquelle l'acide gras constitutif du triacylglycérol est constitué d'un acide gras à chaîne moyenne en C6-10 et d'un acide gras à chaîne longue en C16-24 dans laquelle l'acide gras à chaîne longue en C16-24 est choisi dans le groupe constitué de l'acide palmitique, de l'acide stéarique, de l'acide arachidique, de l'acide béhénique, de l'acide lignocérique, de l'acide palmitoléique, de l'acide oléique, de l'acide linoléique, de l'acide linolénique, de l'acide γ-linolénique, de l'acide arachidonique et de l'acide icosapentaénoïque.

2. Composition d'huile ou de graisse pour une utilisation dans le traitement du diabète pour améliorer l'insulinorésistance selon la revendication 1, dans laquelle l'acide gras à chaîne moyenne est au moins un choisi dans le groupe constitué d'un acide gras saturé comportant 8 atomes de carbone et d'un acide gras saturé comportant 10 atomes de carbone.

3. Aliment ou boisson pour une utilisation dans le traitement du diabète pour améliorer l'insulinorésistance comprenant la composition d'huile ou de graisse selon l'une quelconque des revendications 1 ou 2.
